# EUROPEAN PATENT SPECIFICATION

(11) **EP 2 537 531 B1**
(45) Date of publication and mention of the grant of the patent: **05.11.2014**
(21) Application number: 11744897.7
(22) Date of filing: 17.02.2011
(51) Int. Cl.: A61K 47/44, A61K 31/137, A61P 13/10

(54) **COMPOSITION FOR PERCUTANEOUS ADMINISTRATION OF TOLTERODINE WITH REDUCED SKIN IRRITATION**
ZUSAMMENSETZUNG ZUR PERKUTANEN VERABREICHUNG VON TOLTERODIN MIT REDUZIERTER HAUTREIZUNG
COMPOSITION POUR L'ADMINISTRATION PERCUTANÉE DE TOLTÉRODINE ASSORTIE D'UNE IRRITATION CUTANÉE RÉDUITE

(30) Priority: 18.02.2010 KR 20100014551
(43) Date of publication of application: 26.12.2012
(73) Proprietor: SK CHEMICALS CO., LTD., Suwon-si, Kyungki-do 440-745 (KR)
(72) Inventor: HWANG, Yong Youn, Suwon-si Gyeonggi-do 440-210 (KR); CHOI, Won-jae, Seoul 143-761 (KR); KIM, Jae-Sun, Suwon-si Gyeonggi-do 441-880 (KR); PARK, Yeo-jin, Seoul 131-200 (KR); OH, Joon-gyo, Suwon-si Gyeonggi-do 440-709 (KR); RHEE, Hae-In, Yongin-si Gyeonggi-do 448-918 (KR); IM, Jong-Seob, Suwon-si Gyeonggi-do 440-320 (KR); LEE, Bong-Yong, Seoul 137-060 (KR)
(74) Representative: Peters, Hajo
(86) International application number: PCT/KR2011/001061
(87) International publication number: WO 2011/102655

(56) References cited:
- KR-A- 20070 008 690
- KR-A- 20070 099 703
- KR-A- 20090 024 822
- US-B1- 6 517 864
- AHMED H ELSHAFEEY ET AL: "Utility of Nanosized Microemulsion for Transdermal Delivery of Tolterodine Tartrate: Ex-Vivo Permeation and In-Vivo Pharmacokinetic Studies", PHARMACEUTICAL RESEARCH, KLUWER ACADEMIC PUBLISHERS-PLENUM PUBLISHERS, NL, vol. 26, no. 11, 21 August 2009 (2009-08-21), pages 2446-2453, XP019752626, ISSN: 1573-904X, DOI: 10.1007/S11095-009-9956-5
- ZHAO L ET AL: "Transdermal delivery of tolterodine by O-acylmenthol: In vitro/in vivo correlation", INTERNATIONAL JOURNAL OF PHARMACEUTICS, ELSEVIER BV, NL, vol. 374, no. 1-2, 5 June 2009 (2009-06-05), pages 73-81, XP026108997, ISSN: 0378-5173, DOI: 10.1016/J.IJPHARM.2009.03.005 [retrieved on 2009-03-19]
- PANDIT, V. ET AL.: 'Formulation and Evaluation oftransdermal films for the treatment of overactive bladder' INT. J. PHARMTECH RES. vol. 1, no. 3, 2009, pages 799 - 804, XP008165064

## Description

### TECHNICAL FIELD

The present disclosure relates to a composition for transdermal administration which contains tolterodine as an active ingredient and relieves skin irritation caused by tolterodine.

### BACKGROUND ART

An overactive bladder refers to a disease with symptoms of urinary frequency, urinary urgency or urgent urinary incontinence. Urinary frequency is a need to urinate more than usual, mostly more than eight times a day, and still having the desire to urinate even when the bladder is not full. Urinary urgency is a sudden strong uncontrollable urge to urinate. Urgent urinary incontinence is a condition of involuntary leakage of urine. Excessively frequent contraction of the smooth muscle of the bladder (detrusor muscle) is presumed to be a cause of overactive bladder. That is, the bladder muscle contracts more frequently than normal or when unnecessary, causing a sudden uncontrollable urge to urinate before the bladder is full. Most causes of such bladder muscle contraction remain unknown. In some cases, symptoms of an overactive bladder develop due to problems in neurotransmission from the brain to the bladder or neuronal damage arising from surgery or parturition. For males, overactive bladder diseases may be accompanied by prostatic hyperplasia.

Overactive bladder can lead to lack of sleep, reduced work efficiency, avoidance of sexual life, and depression. Overactive bladder can eventually lead to a poor quality of life due to lack of information concerning overactive bladder and feelings of shame.

U.S. Patent No. 5,382,600 discloses (substituted) 3,3-diphenylpropylamines useful for the treatment of urinary incontinence. Particularly, this patent discloses that 2-[(1R)-3-(diisopropylamino)-1-phenylpropyl)-4-methylphenol, which has a general name of tolterodine and is known as N,N-diisopropyl-3-(2-hydroxy-5-methylphenyl)-3-phenylpropylamine, is useful for the treatment of urinary incontinence. Tolterodine is currently used in the form of an acid addition salt with a pharmaceutically acceptable acid. A tolterodine acid addition salt commercially available for the treatment of overactive bladder is tolterodine tartrate.

Presently, anticholinergic drugs account for most therapeutic drugs for overactive bladder. However, anticholinergic drugs have side effects, such as dry mouth, constipation and disturbance of consciousness, which serve as great barriers to patients with overactive bladder in need of long-term administration. Particularly, the administration of anticholinergic drugs increases the risk of urinary obstruction in patients with prostatic hyperplasia and is prohibited in patients with dementia and patients with glaucoma. Under these circumstances, there is a need to develop a novel therapeutic drug or a pharmaceutical drug delivery system that can improve the quality of life. One approach to reduce the side effects of a therapeutic drug for overactive bladder is to biopharmaceutically control the blood level of the drug above a minimum effective concentration and below a concentration at which the side effects occur. For this approach, studies on sustained-release pharmaceutical drug delivery systems are much underway. Currently commercially available dosage forms of tolterodine are film-coated tablets containing 1 mg, 2 mg or 4 mg of tolterodine L-tartrate that is released in the gastrointestinal tract upon administration.

Transdermal drug delivery systems are technologies by which drugs are absorbed through the skin. Transdermal drug delivery systems have particular advantages of reduced side effects, particularly, in the gastrointestinal tract, bypass of hepatic first pass and enhanced convenience of administration to patients due to their ability to maintain the concentration of drugs at a constant level compared to other drug delivery systems. Therefore, transdermal drug delivery systems can be considered an optimal system to reduce the side effects of therapeutic drugs for overactive bladder and enhance the convenience of administration to patients.

Drugs for use in transdermal delivery systems should meet the following general requirements: 1) molecular weight ≤ 500 Dalton, 2) melting point ≤200 °C, 3) 1 ≤ oil-water partition coefficient ≤ 3, 4) daily dose ≤ 20 mg, and 5) less skin irritation or low sensitivity. Of these requirements, skin irritation or sensitivity is the most important consideration in the selection of drugs for transdermal administration or the development of preparations for transdermal administration. In actuality, some preparations for transdermal administration available in the market have not been commercially successful due to signs of skin irritation such as erythema, edema and itching.

U.S. Patent No. 6,517,864 discloses that transdermal administration of tolterodine is useful for the treatment of overactive bladder. Particularly, this patent discloses that small doses and short half-life make tolterodine favorable as a drug for transdermal administration and tolterodine in the form of free base is more effective in skin penetration than tolterodine in the form of an acid addition salt with a pharmaceutically acceptable acid. However, this patent fails to disclose the disadvantages of tolterodine in terms of skin irritation and sensitivity.

### DISCLOSURE

### Technical Problem

The above U.S. patent discloses that tolterodine causes little to no skin irritation upon transdermal administration. However, as a result of intensive research on preparations for transdermal administration of tolterodine, the inventors of the present disclosure have found that tolterodine free base causes skin irritations including erythema, edema and itching. The present disclosure is designed to solve the problems of the prior art, and therefore it is an object of the present disclosure to provide a pharmaceutical composition for transdermal administration which contains tolterodine and reduces skin irritation caused by tolterodine.

### Technical Solution

In order to achieve the object of the present disclosure, there is provided a composition for transdermal administration which comprises tolterodine as an active ingredient and at least one essential oil (more preferably bisabolol and/or eugenol) as an ingredient for skin irritation relief.

As explained earlier, in the course of research aimed at developing preparations for transdermal administration of tolterodine, the inventors of the present disclosure have found that tolterodine causes irritation to the skin. To solve this problem of skin irritation, the inventors of the present disclosure have investigated and tested tens of species of substances capable of relieving skin irritation caused by tolterodine. As a result, the inventors of the present disclosure have found that certain essential oils can reduce skin irritation caused by tolterodine. The present disclosure has been accomplished based on this finding.

More specifically, the inventors of the present disclosure have collectively reviewed a wide variety of ingredients, including essential oils, gentisic acid, allantoin, stearyl glycyrrhetinate, tocopherol acetate, centella extracts, methyl nicotinate, cinnamyl alcohol, 3-phenyl-1-propanol, 4-phenyl-1-butanol, 2-phenylethanol, KNO₃, K₂CO₃ and Li₂CO₃ in terms of ease of producing preparations for transdermal preparations, adhesiveness to the skin when they are prepared into patch formulations, the solubility of tolterodine, skin permeability, etc., and have particularly reviewed skin irritation relieving effects of these ingredients. As a result of this review, the inventors of the present disclosure have discovered that essential oils are most preferred.

The essential oil used in the composition of the present disclosure is a fragrant volatile oil-soluble liquid substance extracted from a plant. Essential oils have been medically used for aromatherapy since ancient times. Aromatherapy is a kind of natural healing method that is currently attracting attention as alternative medicine.

Examples of essential oils suitable for use in the composition of the present disclosure include limonene, eugenol, geraniol, bisabolol and eucalyptol. Bisabolol and eugenol are even more preferred in terms of skin irritation relief compared to other essential oils.

The composition for transdermal administration according the present disclosure may further comprise lidocaine or a pharmaceutically acceptable salt thereof, in addition to tolterodine as an active ingredient and the essential oil (preferably bisabolol and/or eugenol) as an ingredient for skin irritation relief. The composition comprising tolterodine and the essential oil is satisfactory in reducing erythema and edema, which are representative skin irritations caused by tolterodine, but is relatively weakly effective in reducing itching, which is another representative skin irritation caused by tolterodine, although the exact cause for this is not clearly understood. The combination of the two-ingredient composition with lidocaine has a synergistic effect on the reduction of skin irritations as side effects caused by tolterodine.

In the present disclosure, the essential oil is used in an amount of 1 to 300 parts by weight, preferably 5 to 100 parts by weight, more preferably 10 to 50 parts by weight, based on 100 parts by weight of tolterodine. The use of the essential oil in an amount of less than 1 part by weight is not sufficient in effectively relieving skin irritation caused by tolterodine. Meanwhile, the use of the essential oil in an amount exceeding 300 parts by weight does not contribute to a further improvement in skin irritation relief and disadvantageously causes a reduction in the adhesive strength of a patch in the form of a matrix to be produced.

The composition of the present disclosure may use either tolterodine free base or an acid addition salt of tolterodine. The use of tolterodine free base is more preferred taking into consideration suitable physiochemical properties of the drug for transdermal absorption.

The composition for transdermal administration according to the present disclosure can be prepared into formulations, for example, a patch, a liquid and an ointment. A patch is most preferred.

The patch may use a suitable base known in the art, for example, an excipient assisting in the transdermal absorption of the composition or an adhesive assisting in the adhesion of the composition to the skin. An abirritant may be added to relieve skin irritation so long as the object of the present disclosure is not impaired.

A solvent may be used to dissolve the drug in the course of producing the patch. Examples of such solvents include ethyl acetate, ethanol, isopropanol and propylene glycol. The solvent is typically used in an amount of 50 to 500 parts by weight, based on 100 parts by weight of tolterodine.

The composition for transdermal administration according to the present disclosure may use a pressure sensitive adhesive (PSA) for medical applications as an adhesive polymer when it is prepared into a patch formulation. The adhesive polymer may be based on water or organic solvent. Examples of preferred adhesive polymers include: acrylic adhesives, such as acrylate polymers and vinyl acetate-acrylate copolymers; polyisobutylene; polystyrene; polybutadiene; copolymers thereof, such as synthetic and natural rubbers; and silicone-based adhesives. These adhesive polymers may be used alone or as a mixture of two or more thereof.

The content of the adhesive polymer is preferably from 500 to 5,000 parts by weight, based on 100 parts by weight of tolterodine. If the adhesive polymer is used in an amount of less than 500 parts by weight, a homogeneous phase cannot be formed as a result of mixing with the other additives, making it impossible to produce a patch and causing problems such as reduced adhesive strength. Meanwhile, if the adhesive polymer is used in an amount exceeding 5,000 parts by weight, the relatively low contents of the drug and the other additives preclude the individual ingredients to sufficiently play their roles, resulting in a marked decrease in the absorption of the drug.

The dosage of the composition of the present disclosure may vary depending on the age, weight, sex and health of patients being treated, the mode of administration, and severity of the disease being treated.

### Advantageous Effects

The composition for transdermal administration according to the present disclosure relieves skin irritation caused by tolterodine. Therefore, a successful commercial application of a tolterodine-containing transdermal preparation based on the composition of the present invention can be expected.

### MODE FOR DISCLOSURE

The present disclosure will be explained in detail with reference to the following examples. However, these examples may be embodied in various different forms and should not be construed as limiting the scope of the present disclosure. The examples are provided to more fully explain the present disclosure to those having ordinary knowledge in the art to which the present disclosure pertains.

### EXAMPLES

### <Production Examples 1-2> Production of tolterodine-containing patches

In accordance with the composition shown in Table 1, a solution of tolterodine free base in ethanol was mixed with the acrylic pressure sensitive adhesive for about 10 min. The mixture was allowed to stand at room temperature for at least 4 hr to remove bubbles. The resulting mixture was applied to a thickness of 400 µm onto a silicone-coated polyester film at a rate of 0.5 m/min using an applicator, dried in a hot-air dryer at 80 °C for 15 min, followed by lamination with a polyethylene film to produce a tolterodine-containing patch (Production Example 1). A patch was produced in the same manner as described above, except that tolterodine free base was not used (Production Example 2).

**TABLE 1**

| | Tolterodine free base (g) | Acrylic pressure sensitive adhesive (g) |
|---|---|---|
| Production Example 1 | 1.32 | 12.2 |
| Production Example 2 | 0.00 | 12.2 |

### <Test Example 1> Skin irritation test

Each of the patches produced in Production Examples 1 and 2 was cut to a size of 1 cm × 1 cm. The specimen was attached to the forearm skin of a test subject and fixed with a medical tape to prevent detachment during the test period. After 24 hr, the medical tape and the patch were removed with care. After 1 hr, the primary skin irritation index of the patch for skin irritations including erythema and edema was determined with the help of an expert.

**TABLE 2**

| | Primary skin irritation index |
|---|---|
| Production Example 1: Patch containing tolterodine free base | 0.85 |
| Production Example 2: Patch containing no tolterodine free base | 0.01 |

From the results in Table 2, it was confirmed that tolterodine is a substance causing skin irritations, such as erythema and edema.

### <Production Examples 3-7> Production of patches containing essential oils and tolterodine

In accordance with the compositions shown in Table 3, a solution of tolterodine free base and the essential oil in ethanol was mixed with the acrylic pressure sensitive adhesive for about 10 min. The mixture was allowed to stand at room temperature for at least 4 hr to remove bubbles. The resulting mixture was applied to a thickness of 500 µm onto a silicone-coated polyester film at a rate of 0.5 m/min using an applicator, dried in a hot-air dryer at 80 °C for 15 min, followed by lamination with a polyethylene film to produce a tolterodine-containing patch (Production Examples 4-7). A patch was produced in the same manner as described above, except that no essential oil was used (Production Example 3).

**TABLE 3**

| | Tolterodine free base (g) | Acrylic pressure sensitive adhesive (g) | Essential oil (g) |
|---|---|---|---|
| Production Example 3 | 1.32 | 12.2 | 0.00 |
| Production Example 4 | 1.32 | 12.2 | Bisabolol 1.32 |
| Production Example 5 | 1.32 | 12.2 | Bisabolol 0.66 |
| Production Example 6 | 1.32 | 12.2 | Eugenol 1.32 |
| Production Example 7 | 1.32 | 12.2 | Limonene 0.33 |

### <Test Example 2> Effects of essential oils on skin irritation relief

Skin irritation tests were conducted in the same manner as in Test Example 1. The results are shown in Table 4.

**TABLE 4**

| | Essential oil | Primary skin irritation index |
|---|---|---|
| Production Example 3 | - | 0.58 |
| Production Example 4 | Bisabolol | 0.03 |
| Production Example 5 | Bisabolol | 0.09 |
| Production Example 6 | Eugenol | 0.06 |
| Production Example 7 | Limonene | 0.25 |

The results in Table 4 show that skin irritation caused by tolterodine was relieved by the use of the essential oils. Particularly, bisabolol and eugenol had outstanding effects on skin irritation relief.

## Claims

1. A composition for transdermal administration comprising tolterodine, **characterized in that** the composition comprises an essential oil for skin irritation relief.

2. The composition for transdermal administration according to claim 1, wherein the tolterodine is in the form of free base.

3. The composition for transdermal administration according to claim 1, wherein the essential oil is used in an amount of 1 to 300 parts by weight, based on 100 parts by weight of the tolterodine.

4. The composition for transdermal administration according to claim 1, wherein the essential oil is at least one selected from the group consisting of limonene, eugenol, geraniol, bisabolol, and eucalyptol.

5. The composition for transdermal administration according to claim 4, wherein the essential oil is eugenol, bisabolol or a mixture thereof.

6. The composition for transdermal administration according to any one of claims 1 to 5, further comprising lidocaine or a pharmaceutically acceptable salt thereof.

7. The composition for transdermal administration according to claim 1, wherein the composition comprises 500 to 5,000 parts by weight of an adhesive acrylate copolymer and 1 to 300 parts by weight of the essential oil, based on 100 parts by weight of the tolterodine.

## Patentansprüche

1. Zusammensetzung zur transdermalen Verabreichung, umfassend Tolterodin, **dadurch gekennzeichnet, dass** die Zusammensetzung ein etherisches Öl zur Linderung der Hautreizung umfasst.

2. Zusammensetzung zur transdermalen Verabreichung nach Anspruch 1, wobei das Tolterodin in Form der freien Base vorliegt.

3. Zusammensetzung zur transdermalen Verabreichung nach Anspruch 1, wobei das etherische Öl in einer Menge von 1 bis 300 Gewichtsteilen, bezogen auf 100 Gewichtsteile des Tolterodins, verwendet wird.

4. Zusammensetzung zur transdermalen Verabreichung nach Anspruch 1, wobei es sich bei dem etherischen Öl um mindestens eines aus der Gruppe bestehend aus Limonen, Eugenol, Geraniol, Bisabolol und Eukalyptol handelt.

5. Zusammensetzung zur transdermalen Verabreichung nach Anspruch 4, wobei es sich bei dem etherischen Öl um Eugenol, Bisabolol oder eine Mischung davon handelt.

6. Zusammensetzung zur transdermalen Verabreichung nach einem der Ansprüche 1 bis 5, ferner umfassend Lidocain oder ein pharmazeutisch unbedenkliches Salz davon.

7. Zusammensetzung zur transdermalen Verabreichung nach Anspruch 1, wobei die Zusammensetzung 500 bis 5000 Gewichtsteile eines klebenden Acrylatcopolymers und 1 bis 300 Gewichtsteile des etherischen Öls, bezogen auf 100 Gewichtsteile des Tolterodins, umfasst.

## Revendications

1. Composition destinée à une administration transdermique comprenant de la toltérodine, **caractérisée en ce que** la composition comprend une huile essentielle pour le soulagement des irritations cutanées.

2. Composition destinée à une administration transdermique selon la revendication 1, **caractérisée en ce que** la toltérodine se trouve sous la forme d'une base libre.

3. Composition destinée à une administration transdermique selon la revendication 1, **caractérisée en ce que** l'huile essentielle est utilisée selon une quantité allant de 1 à 300 parties en poids, sur la base de 100 parties en poids de la toltérodine.

4. Composition destinée à une administration transdermique selon la revendication 1, **caractérisée en ce que** l'huile essentielle est au moins une huile choisie dans le groupe constitué par le limonène, l'eugénol, le géraniol, le bisabolol et l'eucalyptol.

5. Composition destinée à une administration transdermique selon la revendication 4, **caractérisée en ce que** l'huile essentielle est l'eugénol, le bisabolol, ou un mélange de ceux-ci.

6. Composition destinée à une administration transdermique selon l'une quelconque des revendications 1 à 5, comprenant en outre de la lidocaïne ou un sel pharmaceutiquement acceptable de celle-ci.

7. Composition destinée à une administration transdermique selon la revendication 1, **caractérisée en ce que** la composition comprend de 500 à 5000 parties en poids d'un copolymère d'acrylate adhésif et de 1 à 300 parties en poids de l'huile essentielle, sur la base de 100 parties en poids de la toltérodine.
